# EUROPEAN PATENT APPLICATION

(11) **EP 1 676 818 A1**
(43) Date of publication of application: **05.07.2006**
(21) Application number: 05020482.5
(22) Date of filing: 20.09.2005
(51) Int. Cl.: C02F 9/00, C02F 1/00, A61L 2/10, C02F 1/24

(54) **Filtering and purifying system**

(30) Priority: 04.01.2005 JP 2005000020
(71) Applicant: HITACHI, LTD., Chiyoda-ku Tokyo 100-8280 (JP)
(72) Inventor: Saho, Norihide Hitachi, Ltd., Tokyo 100-8220 (JP); Imamura, Yoichi Hitachi, Ltd., Tokyo 100-8220 (JP); Mochizuki, Akira Hitachi, Ltd., Tokyo 100-8220 (JP); Isogami, Hisashi Hitachi, Ltd., Tokyo 100-8220 (JP); Mizumori, Takashi Hitachi, Ltd., Tokyo 100-8220 (JP)
(74) Representative: Beetz & Partner

(57) **Abstract**

The invention relates to a filtering and purifying system in which bacteria in raw water (2) are sterilized efficiently with ultraviolet rays (74), chemicals and the like at a pretreating step. The number of microorganisms such as bacteria remaining in treated water (17) is reduced remarkably, and the residue of plankton and organic substances as nutrients for bacteria in the treated water is reduced by a coagulating (5, 7, 9, 11, 13, 15) and filtering (19) treatment. It is thus possible to solve a problem that the treated water is deteriorated with time due to propagation of the bacteria.

## Description

### BACKGROUND OF THE INVENTION

The present invention relates to a filtering/separating and purifying system for purification of water and for solid-liquid separation, and particularly, to the construction of the filtering and purifying system capable of reducing the number of microorganisms such as bacteria remaining in treated water.

There is a magnetically separating and purifying system in which a fine metal net or a net knitted of polymer fibers is used as a through-flow separating membrane for the purpose of solid-liquid separation, and a flocculating agent and a magnetic powder are added to raw water containing polluting particles to be separated to thereby produce magnetic flocs. The magnetic flocs are then separated by the membrane, the magnetic flocs collected by the membrane are magnetically separated off and removed using a magnetic field-generating means, and a high-concentration sludge is recovered.

This construction is described, for example, in JP-A-2002-273261. The filtering/separating and purifying system includes a membrane separating section comprising a net formed of fine stainless steel wires or polyester fibers and having openings of an opening size of, for example, several tens micrometers. To separate a fine polluting material smaller than a projected area and a projected diameter.of the openings, for example, alumina sulfate, aluminum polychloride or iron polysulfate as a flocculating agent and a magnetic powder are previously added to raw water and stirred, and a fine solid suspended matter, algae and microorganisms in the raw water are coagulated into a size on the order of several hundred micrometers by the flocculating agent to form magnetic flocs. The magnetic flocs can not pass through the openings having the opening size of several tens micrometers, and are separated and caught with a high removal rate. The water penetrated through the membrane is purified water having a high quality with the residue of the fine solid suspended matter, algae and microorganisms in the raw water being several percents.

The magnetic flocs caught on the membrane are washed away from the membrane by washing water, and thereafter, the magnetic flocs stagnating in the vicinity of water surface are attracted and magnetically separated by the magnetic force of a magnet disposed stationary in the vicinity of the water surface, and then transferred to a sludge recovery tank and eliminated by a sludge transfer means. Finally, the sludge may be burned off on a land or on a sea, or may be composted.

According to the above Patent document, bacteria having vigorous propagating power particularly under appropriate surrounding conditions, e.g., microorganisms such as colon bacilli remain in treated water at several percents of those in raw water, and colon bacilli propagate in a short time under the appropriate surrounding conditions. In a purifying system for life waste water, for example, in a ship in which the treated water is stored for a given period, the water is purified during voyaging of the ship in order to meet an effluent standard for life waste water, but there is a problem that for the period of storage of the treated water in a life waste water tank, colon bacilli in the treated water propagate for a voyaging period to deteriorate the quality of the treated life waste water beyond the effluent standard and as a result, discharging of the treated water becomes unacceptable.

### BRIEF SUMMARY OF THE INVENTION

It is an object of the present invention to provide a filtering and purifying system capable of reducing the concentration of residual possible nutrients to remarkably reduce the propagating function of bacteria.

The above object is achieved by a filtering and purifying system comprising a production means for producing a product by coagulating matter to be removed in a fluid to be treated, chemically catching and bonding the matter, and/or a filtering means having an opening size through which the product provided by the production means at a size larger than that of the matter to be removed can not pass. A treating means for at least sterilizing or oxidizing the matter to be removed in the fluid to be treated is provided in the production means.

The above object can also be achieved by a filtering and purifying system comprising a fluid storing means for storing a fluid to be treated, a production means for producing a product by coagulating matter to be removed in the fluid to be treated, chemically catching and bonding the matter, and/or a filtering means having an opening size through which the product provided by the production means at a size larger than that of the matter to be removed can not pass. A treating means for at least sterilizing or oxidizing the matter to be removed in the fluid to be treated may be provided in the fluid storing means.

Further, the above object can be achieved by a filtering and purifying system comprising a production means for producing a product by coagulating matter to be removed in a fluid to be treated, chemically catching and bonding the matter, a filtering means having an opening size through which the product provided by the production means at a size larger than that of the matter to be removed can not pass, and/or a treated-water storing means for storing treated water filtered by the filtering means. A treating means for at least sterilizing or oxidizing the matter to be removed in the fluid to be treated can be provided in the treated-water storing means.

Furthermore, the above object can be achieved by a filtering and purifying system comprising a production means for producing a product by coagulating matter to be removed in a fluid to be treated, chemically catching and bonding the matter, and/or a filtering means having an opening size through which the product provided by the production means at a size larger than that of the matter to be removed can not pass. The production means can have therein a fluid stirring means for stirring the fluid to be treated, and a treating means for at least sterilizing or oxidizing the matter to be removed in the fluid to be treated is provided in the fluid stirring means.

Yet further, the above object can be achieved by a filtering and purifying system comprising a production means for producing a product by coagulating matter to be removed in a fluid to be treated, chemically catching and bonding the matter, and/or a filtering means having an opening size through which the product provided by the production means at a size larger than that of the matter to be removed can not pass through the filtering means. A plurality of treating means for at least sterilizing or oxidizing the matter to be removed in the fluid to be treated and a restoring means for restoring reduced sterilizing or oxidizing functions of the treating means can be provided in the production means, and/or at least one or more of the treating means with the sterilizing or oxidizing functions restored function continuously during purifying operation.

According to the invention, it is possible to provide the filtering and purifying system capable of reducing the concentration of residual possible nutrients and remarkably reducing the propagating function of bacteria.

Other objects, features and advantages of the invention will become apparent from the following description of the embodiments of the invention taken in conjunction with the accompanying drawings.

### BRIEF DESCRIPTION OF THE SEVERAL VIEWS OF THE DRAWING

Fig. 1 is a flow diagram of the filtering and purifying system according to an embodiment of the invention.

Fig. 2 is a sectional view of a magnetically separating section in the embodiment of the invention.

Fig. 3 is a sectional view taken along a line A-A in Fig. 2.

Fig. 4 is a flow diagram of the filtering and purifying system according to another embodiment of the invention.

Fig. 5 is a view for explaining a further embodiment of the present invention.

Fig. 6 is a view for explaining a still further embodiment of the invention.

### DETAILED DESCRIPTION OF THE INVENTION

Embodiments of the invention will be now described with reference to the drawings.

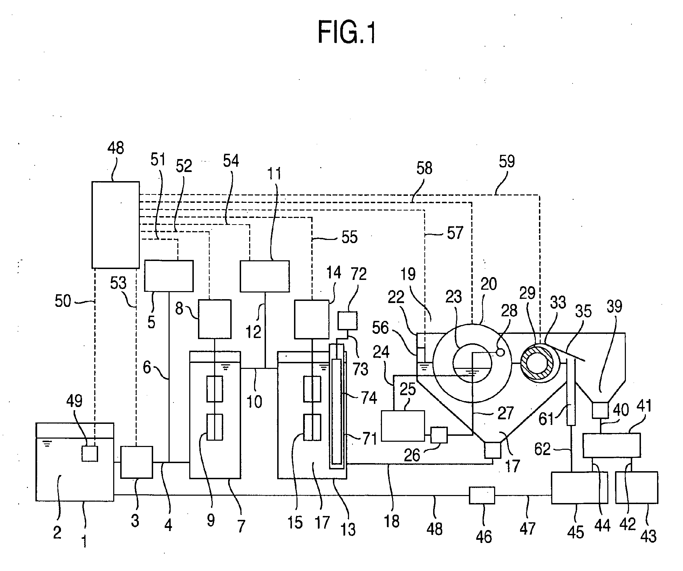

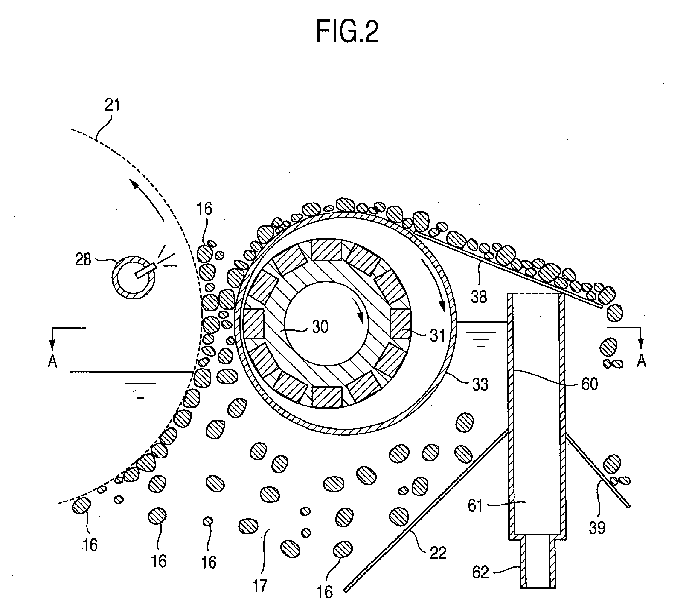

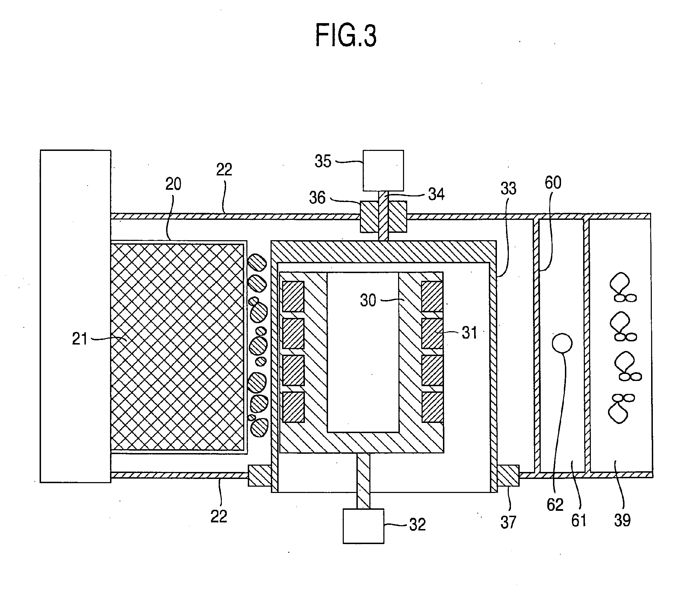

The first embodiment of the invention will be described with reference to Figs. 1, 2 and 3. Fig. 2 is an enlarged sectional view of a membrane separator 14 shown in Fig. 1, and Fig. 3 is a sectional view taken along a line A-A in Fig. 2.

Raw water 2 which is water to be treated, for example a life waste water introduced into a ship at a port visit, and from which large refuse of several millimeters has been removed, is stored in a raw water storage tank 1, for example a life waste water tank in the ship, and the raw water 2 is fed in a predetermined amount to a pipe line 4 by a pump 3. A magnetic powder such as iron tetroxide, a pH adjuster, a flocculating agent such as an aqueous solution of aluminum polychloride, ferric chloride or ferric sulfate which provides aluminum ion or iron ion, a polymer reinforcing agent and the like are supplied from a seeding agent adjusting device 5 through a conduit 6 into the pipe line 4 and then stirred at a high speed in a stirring tank 7 by a stirring blade 9 driven for rotation by a motor 8, thereby producing magnetic micro flocs of several hundred micrometers.

Thereafter, a polymer reinforcing agent or the like is supplied from a polymer agent adjusting device 11 through a conduit 12 into a pipe line 10 and then stirred slowly at a low speed by a stirring blade 15 driven for rotation by a motor 14 in a stirring tank 13, whereby the magnetic micro-floc groups are entangled and agglomerated by the polymer reinforcing agent to produce a pretreated water 17 containing magnetic flocs 16 (not shown in Fig. 1) having a size on the order of several millimeters.

A transparent tube for allowing passage of ultraviolet rays therethrough from the side of the atmosphere, for example a glass tube 74, is immersed into the pretreated water in the stirring tank 13, and a sterilizing ultraviolet lamp 74 connected to a power source 72 by a wire 73 is inserted into the glass tube 71 to apply ultraviolet rays into the pretreated water. In the stirring tank 13, the pretreated water is mixed all over by the stirring blade 15 and hence, the ultraviolet rays are applied even to bacteria, e.g., colon bacilli, on the back side of the produced flocs as viewed from the side of the ultraviolet lamp as the flocs move, whereby the pretreated water is sterilized. Here, the pretreated water is mixed for several minutes and hence, is constantly irradiated with the ultraviolet rays. In the stirring tank 13, polluted particles are taken into the magnetic flocs, whereby the transparency of the pretreated water is increased, and the ultraviolet rays from the ultraviolet lamp are transmittable through the entire region of the pretreated water. Therefore, most of the colon bacilli which have not been taken into the flocs are killed, and the living colon bacilli in the treated water are remarkably decreased. The pretreated water is moved all over to the vicinity of the ultraviolet lamp by the stirring blade and hence, the ultraviolet lamp having a weaker illumination intensity and a smaller power consumption can be used, leading to an effects of reducing the lamp cost and a sterilizing operational cost.

The pretreated water 17 thus produced is passed through a conduit 18 into a membrane separator 19. The structure of the membrane separator 19 will be described below with reference to Figs. 2, 3. A net 21 serving as a membrane with openings having an opening size of from several micrometers to several ten micrometers and made of a small-gage wire of stainless steel, a small-gage wire of copper, polyester fibers or the like is mounted on an outer peripheral surface of a rotary drum 20 shown in Fig. 3. The pretreated water flows into a water tank 22 and is passed through the net 21 into the drum 20. At this time, the magnetic flocs 16 in the pretreated water are caught on an inner surface of the net 21, and the water passed through the net 21 with the magnetic flocs separated therefrom is discharged in the form of purified water through an opening 23 shown in Fig. 1, passed through a pipe line 24, accumulated in the washing water tank 25, and discharged to outside the system or stored in the life waste water tank in the ship. A power for the pretreated water to pass through the net 21 is a difference in surface level between the pretreated water 17 and the purified water in the drum 20.

The colon bacilli in the pretreated water which could not be filtered in the net 21 remain in the treated water, but most of the colon bacilli are dead. Bacteria and organic substances having a size equal to or larger than 0.1 µm in the life waste water have been taken into the magnetic flocs and little remain in the treated water filtered by the net 21. Thus, as there is only little living colon bacilli remaining in the treated water, and as the amount of nutriments in the treated water has been remarkably decreased, it is possible to substantially prevent the living colon bacilli from being proliferated within a period of storage of the treated water. Therefore, the treated water is note deteriorated, may maintain the water quality which met the discharge water standard at the time of the treatment, and may be discharged to the outside of the ship even after being stored.

The magnetic flocs 16 are filtered by the net 21 and deposited on its outer surface rotated in a counterclockwise direction as viewed in Fig. 2, and exposed in the form of a deposit to the atmosphere above the liquid surface. The purified water in the washing water tank 25 of Fig. 1 is pressurized and fed through a conduit 27 to a shower pipe 28 by a pump 26, and shower water is sprayed from apertures to the outer surface of the net 21 from the inner surface side of the net 21. The magnetic flocs 16 accumulated on the outer surface of the net 21 are peeled off by the shower water, and the surface of the net 21 is regenerated. The magnetic flocs washed off stay on the surface of the pretreated water 17 in the water tank 22.

A rotary magnet 29 (Fig. 1) used as a magnetic field generating means for the magnetic separation has a construction in which a plurality of permanent magnet elements 31 are fixed by an adhesive or the like into a plurality of grooves in an outer surface of a rotor 30 (Fig. 2) made of a non-magnetic material and the rotor 30 is rotated at a controlled rotational speed by a motor 32 (Fig. 3).

On the other hand, as shown in Fig. 3, a sludge transferring rotor 33 made of a non-magnetic material and used for transferring the magnetic flocs magnetically separated off is rotated at a control rotational speed through a shaft 34 by a motor 35. At one end, the shaft 34 is supported on a wall of the water tank 22 by a rotary support 36 having a watertightness, and at the other end, an outer periphery of the rotor 33 is supported on the wall of the water tank 22 through a rotary support 37 also having a watertightness, wherein the inside of the rotary support 37 is opened to the atmosphere. The magnet 29 shown in Fig. 11 is inserted into the inside of the rotor 33 from the side of the rotor 33 opened to the atmosphere, and is placed in proximity of a location where the magnetic floc 16 groups washed off by washing water are staying, i.e., a location closer to the rotary drum.

In this embodiment, the rotors 33 and 30 are arranged with their axes offset from each other. Although not shown in the figures, the magnet 29 is fixed to a portion of the water tank 22 by bolts or the like, so that it is positioned at a predetermined location. The rotational directions of the rotors 33 and 30 are the same, and the rotors 33 and 30 are rotated in the direction for moving the magnetic floc groups magnetically attracted thereto toward the atmosphere side. The numbers of rotation of the rotors 33, 30 may be the same or different. In the case of this embodiment, the rotation speed of the rotor 30 on the magnet side is larger than that of the rotor 33.

The magnetic floc 16 groups washed down and staying in the vicinity of the water surface are attracted and moved toward the magnet side by the magnetic field of the magnet 29, attach to the outer surface of the rotor 33 rotated outside the magnet 29, and thereafter are exposed to the atmosphere with the rotation of the rotor 33. A surplus amount of water in the magnetic floc 16 groups flows down the surface of the rotor 33 by gravitation, and the magnetic floc 16 groups are further concentrated. Here, the water content of the magnetic flocs is lowered to about 97%.

The magnetic floc groups concentrated on the surface of the rotor 33 are moved by the rotation of the rotor 33. At this time, the axes of the rotors 30, 33 get gradually away from the magnet 29, since they are misaligned from each other, whereby the magnetically attracting force is rapidly decreased as they are more apart from the magnet. The magnetic floc 16 groups are peeled off from the surface of the rotor 33 by a spatula 38 supported on a portion of the water tank 22 to scrape off them, drop into a sludge recovery tank 39 by gravitation, and are collected as a sludge.

The sludge discharged is introduced through a pipe line 40 into a dewatering device 41 such as a centrifugal separator, a belt press or the like, where the sludge is concentrated into a water content equal to or lower than about 85 % enough to prevent water to be leaked from the sludge during transportation of the sludge, or to a water content of a bout 75 % enough to permit the activation of microorganisms for decomposing organic substances at the time of composting. The sludge of a high concentration is fed through a pipe line 42 into a sludge tank 43 and stored.

Treated sewage dewatered in the dewatering device is fed through a pipe line 44 into a sewage treating tank 45, pressured by a pump 46, then returned through a pipe line 47 to the raw water tank 1, and introduced again into the pretreating step. With regard to an operation control unit 48, a surface level, turbidity, temperature, a pH value and the like of the raw water are detected by a sensor 49, and the information is transmitted through a signal line to the operation control unit 48. Amounts of chemicals (the pH adjuster, the magnetic powder, the flocculating agent) to be added, which are optimal to produce good magnetic flocs, are calculated based on the information using an optimal amount calculating program previously inputted, and the resulting control information is transmitted via a signal line 51 to a chemical agent tank 5 for addition of the optimal amounts.

Further, a number of rotation of the stirring motor and a time period of staying in the stirring tank are calculated in the operation control unit 48, and the resulting control information is transmitted via a signal line 52 to the motor 8 to rotate the stirring blade 9 at the optimum rotation speed and is transmitted via a signal line 53 to control a discharge rate of the pump 3 which decides the staying time in the stirring tank. Furthermore, an adding amount of a chemical (high molecular polymer) optimal to produce good magnetic flocs is calculated with the optimal amount calculating program previously inputted, and the resulting control information is transmitted via a signal line 54 to the chemical agent tank 11 to add the optimum amount. At the same time, a number of rotation of the stirring motor is calculated in the operation control unit 48 and transmitted via a signal line 55 to the motor 14 to rotate the stirring blade 15 at the optimum rotation speed.

In the membrane separator 19, on the other hand, a liquid level of the pretreated water 17 in the water tank 22 is detected by a sensor 56, and the information is transmitted via a signal line 57 to the operation control unit 48. An optimum number of rotation of the rotary drum 20 and an appropriate rate of recovering the magnetic floc 16 groups are calculated based on the information using the optimal amount calculating program previously inputted, so that the liquid level of the pretreated water is positioned at a substantial central point of the location of placement of the magnet 29, i.e., a point at which the average value of the magnetic field generated by the magnet 29 is maximum, and the resulting control signals are transmitted via a signal line 58 to a motor (not shown) for rotating the rotary drum and via a signal line 59 to the motor 35, thereby controlling the motors at the optimum rotation speeds.

As can be seen from the above description, as a result of purifying the raw water such as the life waste water with the purifying system of this embodiment, the colon bacilli in the pretreated water which could not be filtered by the filter pass into the purified, treated water, but most of the colon bacilli are dead. In addition, bacteria and organic substances of a size equal to or larger than 0.1 µm in the life waste water have been taken into the magnetic flocs and hence, little remain in the treated water filtered by the filter. Therefore, the living colon bacilli scarcely remain in the treated water, nutriments in the treated water have been remarkably decreased and hence, it is possible to substantially prevent the living colon bacilli from being proliferated within a period of storage of the treated water. Thus, the quality of the treated life waste water is not deteriorated, and the effluent standard for the water quality satisfied at the time of the treatment can be maintained, leading to an effect that the treated water can be stored and then discharged to the outside of the ship.

In this embodiment, the glass tube 71 transparent to permit the transmission of ultraviolet rays therethrough from the atmosphere is inserted into the pretreated water in the stirring tank 13, and the sterilizing ultraviolet lamp 74 is inserted into the glass tube 71, whereby the ultraviolet rays can be emitted into the treated water. In the stirring tank 13, the pretreated water is mixed all over by the stirring blade 15. Therefore, the ultraviolet rays are applied even to colon bacilli on the back side of the produced flocs as viewed from the side of the ultraviolet lamp with the movement of the flocs, and the colon bacilli are killed. The mixing in the stirring tank 13 is conducted for a period of several minutes, and during this time, the ultraviolet rays are constantly applied to the pretreated water. Therefore, the sterilizing time is sufficient, a sterilizing effect is produced sufficiently even by the ultraviolet lamp having a smaller illumination intensity, and thus, most of the colon bacilli which have not been taken into the flocs are killed, leading to an effect of remarkable decreasing of the number of the living colon bacilli in the treated water.

Although the ultraviolet lamp 74 is disposed in the stirring tank 13 in this embodiment, it may be disposed in the stirring tank 7, the membrane separator 19 or the washing water tank 25, and even in this case, a similar effect is obtained.

In this embodiment, when the outer surface of the glass tube 71 is fouled by the treated water, resulting in a reduction in transmission of the ultraviolet rays, then the transmission can be restored by taking the glass tube 71 out of the stirring tank 13, washing and removing the dirt. The washing and removal may be carried out automatically in the stirring tank 13, although not shown in the figures.

The glass tube 71 and the ultraviolet lamp may be disposed in any other place as long as they do not interfere with the rotation of the stirring blade, and a plurality of glass tubes may be disposed between stirring blades, although not shown in the figures. In addition, a plurality of types of ultraviolet lamps having different frequencies and wavelengths effective for a plurality of microorganisms respectively may be disposed, although not shown in the figures.

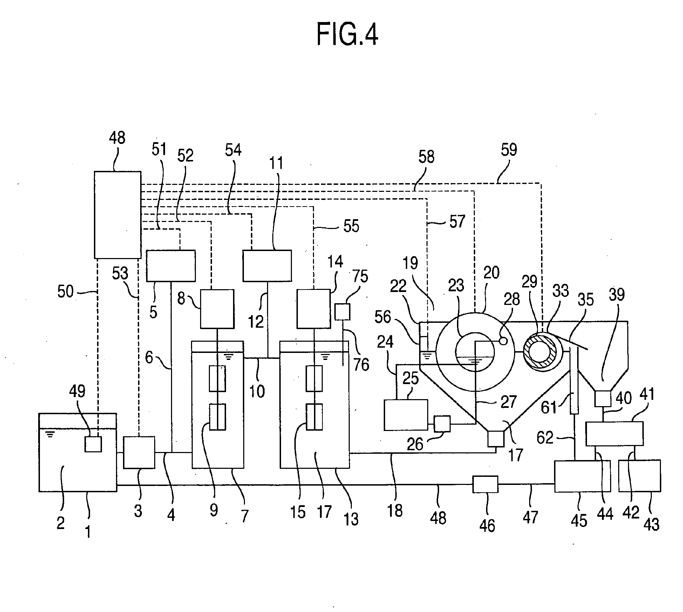

The second embodiment of the invention is shown in Fig. 4. This figure is different from Fig. 1 in that in place of the ultraviolet lamp 74 provided for sterilizing the colon bacilli, a predetermined amount of a chemical in a chemical agent tank 75, for example the chemical for producing hydrogen peroxide, is added through a pipe line 76 into the stirring tank 13. Hydrogen peroxide is produced in the pretreated water in the stirring tank 13, by the power of active oxygen, colon bacilli which have not been taken into the magnetic flocs in the pretreated water are killed and very fine organic substances are oxidized and decomposed.

According to this embodiment, the mixing in the stirring tank 13 is conducted for a period of several minutes and during this time, the pretreated water is constantly mixed. The hydrogen peroxide is therefore spread all over in the pretreated water with no unevenness of concentration caused. Further, there is a sufficient sterilizing time. Therefore, even if the amount of chemical added is controlled to the minimum, a sufficient sterilizing effect is obtained, and most of the colon bacilli in the pretreated water which have not been taken into the flocs are killed, leading to an effect of remarkable decreasing of the number of the living colon bacilli in the treated water.

In this embodiment, parts of vary fine organic substances which have not been taken into the magnetic flocs in the pretreated water are oxidized and decomposed by the oxidizing force of the hydrogen peroxide. Therefore, the residual amount of the organic substances in the treated water as nutriments for the bacteria is further decreased, leading to a further effect of preventing proliferation of the living colon bacilli in the treated water.

In this embodiment, the sterilizing chemical is added into the treated water in the stirring tank 13, it may be added into the raw water in the raw water tank 1, into the pretreated water in the stirring tank 7, into the pretreated water or the treated water in the membrane separator 19, or into the treated water in the washing water tank 25, and in this case, a similar effect is obtained.

Although the above description has been made on the cases where the bacterium-killing treatment and the organic substance-oxidizing treatment have been carried out by providing the ultraviolet lamp and by pouring the chemical from the sterilizing agent tank, substances functioning for sterilizing and oxidizing may be produced or added by providing any suitable device other than the ultraviolet lamp, such as an ozone generating device, an electrolytic hypochlorite-generating device or a ultrasonic wave-generating device, and in this case, a similar effect is obtained.

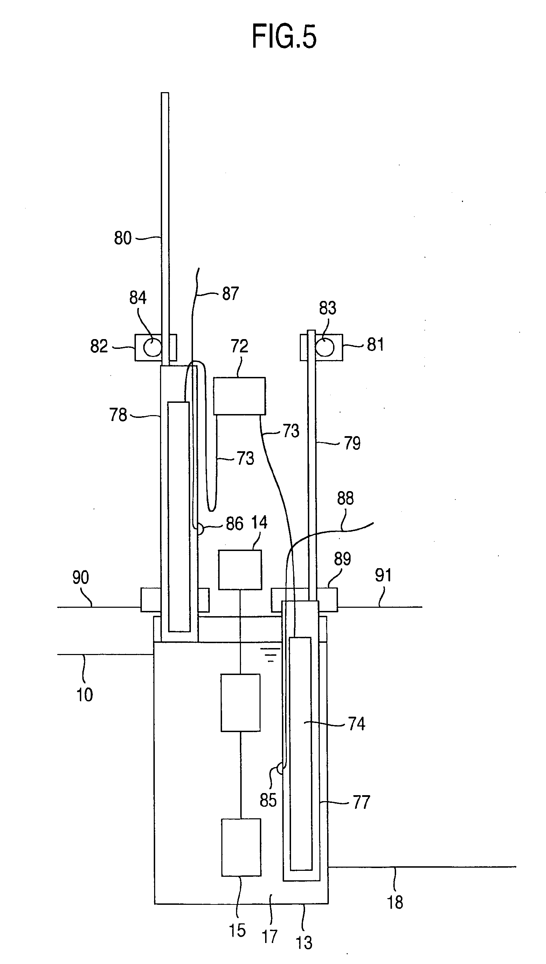

The third embodiment of the invention is shown in Fig. 5. Difference in this figure from the structure of the stirring tank 13 in Fig. 1 is an arrangement in which a plurality of glass tubes each having a colon bacillus-killing ultraviolet lamp 74 therein are provided, and each glass tube is equipped with an elevator for moving it upwards and downwards between a location in the water and the water surface.

The glass tubes 77, 78 with the colon bacillus-killing ultraviolet lamp 74 mounted therein are provided within a stirring tank 13 shown in the figure. The glass tubes 77, 78 are supported by rods 79, 80, respectively, which are movable between positions in the pretreated water in the stirring tank 13 and positions in the atmosphere above the water surface by elevators 81, 82, respectively. The movement of the rods 79, 80 is controlled by controlling the normal and reverse rotation of, for example, gears 83, 84 in the elevators 81, 82.

An outer surface of the glass tube 78 arranged within the stirring tank 13 shown in the figure may be fouled by the pretreated water after operation for a long period of time, thereby decreasing its transmission for ultraviolet rays and reducing the sterilizing performance for killing bacteria in the pretreated water around an outer periphery of the glass tube 78. Ultraviolet ray-transmission sensors 85, 86 for detecting a reduction in the transmission of the ultraviolet rays are mounted on the outer peripheries of the glass tubes 77, 78 at small distances apart from the glass tubes.

When the dirt of the outer surfaces of the glass tubes is detected by the ultraviolet ray-transmission sensor 85, 86, this is transmitted through signal lines 87, 88 to a control unit (not shown), and the fouled glass tubes are moved toward the atmosphere above the water surface by the elevators 81, 82. Fig. 5 shows the case where the glass tube 78 has been moved.

The fouling of outer surfaces, i.e., light-receiving surfaces of the ultraviolet ray-transmission sensors 85, 86 is removed automatically by small wipers (not shown) or the like, respectively.

The outer surface of the glass tube 78 is washed automatically during its movement by a washer 89 which is adapted to wash the glass tube outer surface, for example, with water or an acidic washing liquid and a brash, and a waste liquid after the washing is returned through a pipe line 90 to the raw water tank 1. During this movement, the ultraviolet lamp 74 is turned off.

The glass tube 78 washed stands by as it is, and after the outer surface of the glass tube 77 is fouled and a predetermined level of fouling is detected by the ultraviolet ray-transmission sensor 85, the glass tube 78 is replaced for the glass tube 77 and inserted into the stirring tank 13, and the ultraviolet lamp is turned on.

On the other hand, the glass tube 77 is moved upwards toward the atmosphere with the outer surface of the glass tube 77 being automatically washed during the movement, and the waste water resulting from the washing is returned through a pipe line 91 to the raw water tank 1.

According to this embodiment, any of the ultraviolet lamps 74 in the glass tubes, may be used to continuously kill bacteria in the pretreated water 17, having an effect that the pretreated water can be sterilized without stopping the mixing operation of the stirring tank 13.

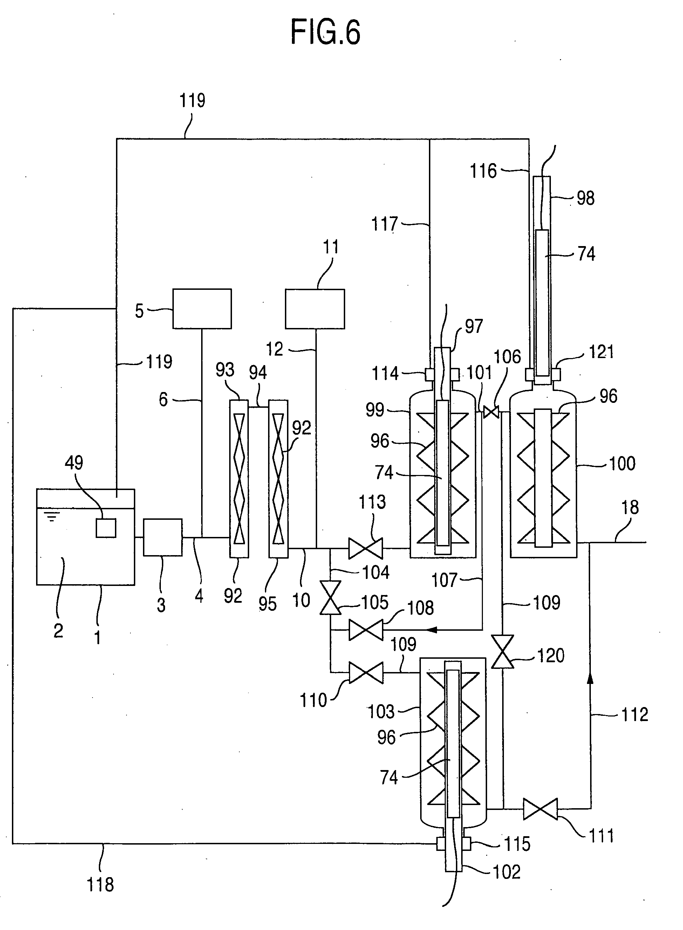

The fourth embodiment of the invention is shown in Fig. 6. Difference in this figure from the structures of the stirring tanks 7, 13 in Fig. 1 is arrangements which will be described below. The blade-type stirring tank 7 is replaced by the arrangement comprising a mixing tube 93 having a ribbon-shaped turbulent flow promoting plate 92 mounted in a flow passage, a pipe line 94 and a mixing tube 95, and the blade-type stirring tank 13 is replaced by the arrangement comprising mixing tubes 99, 100 which are in communicate with each other through a pipe line 101 and which are each equipped in flow passages with doughnut-type ribbon-shaped turbulent flow promoting plates 96 and glass tubes 97, 98 each having a ultraviolet lamp 74 mounted therein.

In addition, as a standby for washing the glass tubes, a mixing tube 103 equipped, in its flow passage, with a doughnut-type ribbon-shaped turbulent flow promoting plate 96 and a glass tube 102 having a ultraviolet lamp 74 mounted therein, is provided in parallel. This is put in communication with the other mixing tubes through a pipe line 104 and valves 105, 106 as well as a pipe line 107 and a valve 108, and further connected to a pipe line 10 and a pipe line 18 through a pipe line 109 and a valve 110 as well as a valve 111 and a pipe line 112.

The turbulent flow promoting plates 92, 96 in the mixing tubes mix and stir the pretreated water within the mixing.tubes with an effect similar to that provided by the stirring blade within the mixing tank, and bacteria in the pretreated water are killed in a manner similar to that in the mixing tank 13 by the application of ultraviolet rays from the ultraviolet lamps 74 in the mixing tubes 99, 100 and 103. The turbulent flow promoting plates 96 provided in the mixing tubes 99, 100 and 103 are stationary, and flowing fluid is stirred and mixed by the disturbance provided by the ribbon-shaped turbulent flow promoting plates.

The outer surfaces of the glass tubes 97, 98 and 102 disposed in the mixing tubes 99, 100 and 103 are fouled with the operation and need washing. Fig. 16 shows a case where the glass tube 98 within the mixing tube 100 is washed.

In this case, the valve 113 in the pipe line 10 is opened, the valve 105 is closed, the valve 108 is opened, the valve 106 is closed, the valve 110 is closed, and the valve 111 is opened. In this case, the pretreatment is carried out by the mixing tubes 99, 103. The glass tube 98 in the mixing tube 100 which is to be washed is moved by an elevator (not shown), and the outer surface of the glass tube 98 is washed automatically by a washer 121 having air-tightness. The mixing tubes 99, 103 are provided with washers 114, 115, respectively. Washing water flows through pipe lines 116, 117, 118 and 119 back to the raw water tank 1.

After the washing of the outer surface of the glass tube 98, it is moved into the mixing tube 100 by the elevator (not shown), the ultraviolet lamp is turned on, the valves 108, 110 and 111 are closed, while the valve 106 is opened, and the ultraviolet lamp 74 in the mixing tube 103 is turned off.

When the glass tube 97 in the mixing tube 99 is to be washed, the valves 113 and 111 are closed, while the valves 105, 110 and 120 are opened, the ultraviolet lamp 74 in the mixing tube 103 is turned on, while the ultraviolet lamp 74 in the mixing tube 99 is turned off, the glass tube 97 is moved by an elevator (not shown), and the outer surface of the glass tube 97 is washed automatically by the washer 114 having air-tightness.

After the washing of the glass tube 97, it is moved into the mixing tube 99 by the elevator (not shown), the ultraviolet amp is tuned on, the valves 113 and 106 are opened, while the valves 105, 110 and 120 are closed, and the ultraviolet amp 74 in the mixing tube 99 is turned off. To wash the glass tube 102 in the standby mixing tube 103, the valves 105, 108, 110 and 111 are closed, the ultraviolet amp 74 in the mixing tube 103 is turned off, the glass tube 102 is moved by an elevator (not shown), and the outer surface of the glass tube 102 is washed automatically by the washer 115 having air-tightness. After the washing, the glass tube 102 is moved into the mixing tube 103 by the elevator (not shown).

In this embodiment, the pretreated water is mixed all over in meandering flows generated by the turbulent flow promoting plates in the mixing tubes. Therefore, ultraviolet rays from the ultraviolet lamps in the central glass tubes are applied all over to the pretreated water during the mixing and stirring, and the bacteria in the pretreated water are killed reliably.

This embodiment has been described above as using the doughnut-type ribbon-shaped plate as the turbulent flow promoting plate, but porous plates may be placed as the turbulent flow promoting plates at a predetermined distance in a direction of flowing of the pretreated water. Also in this case, a similar effect is obtained.

According to this embodiment, the ultraviolet lamps 74 are placed in the mixing tubes and can be used to continuously kill the bacteria in the pretreated water 17. Therefore, even when a mixing tube is used and the outer surface of a glass tube having a infrared lamp mounted therein is fouled, there is an effect that the pretreated water can be sterilized without stopping the mixing in the mixing tube. Although the above embodiments have been described on the case where the water to be purified is the life waste water in the ship, the water to be purified may be ballast water containing plant plankton or animal plankton or bacteria such as cholera germ, colon bacilli and intestine micrococcus and even in this case, a similar effect is obtained.

It should be further understood by those skilled in the art that although the foregoing description has been made on the embodiments of the invention, the invention is not limited thereto and various changes and modifications may be made without departing from the spirit of the invention and the scope of the appended claims.

## Claims

1. A filtering and purifying system comprising a production means (5, 7, 9, 11, 13, 15) for producing a product (16) by coagulating matter to be removed in a fluid (2) to be treated, chemically catching and bonding the matter, and a filtering means (19, 20, 21) having an opening size through which the product (16) provided by the production means at a size larger than that of the matter can not pass, **characterized in that**
a treating means (74; 75) for at least sterilizing or oxidizing the matter to be removed in the fluid to be treated is provided in said production means (13).

2. A filtering and purifying system comprising a fluid storing means (7, 13) for storing a fluid (2) to be treated, a production means (5, 9, 11, 15) for producing a product (16) by coagulating matter to be removed in the fluid to be treated, chemically catching and bonding the matter, and a filtering means (19, 20, 21) having an opening size through which the product (16) provided by the production means at a size larger than that of the matter can not pass, **characterized in that**
a treating means (74; 75) for at least sterilizing or oxidizing the matter to be removed in the fluid to be treated is provided in said fluid storing means (13).

3. A filtering and purifying system comprising a production means (5, 7, 9, 11, 13, 15) for producing a product (16) by coagulating matter to be removed in a fluid (2) to be treated, chemically catching and bonding the matter, a filtering means (19, 20, 21) having an opening size through which the product (16) provided by the production means at a size larger than that of the matter can not pass, and a treated-water storing means (99, 100, 103) for storing treated water (17) filtered by the filtering means, **characterized in that**
a treating means (74) for at least sterilizing or oxidizing the matter to be removed in the fluid to be treated is provided in said treated-water storing means (99, 100, 103).

4. A filtering and purifying system comprising a production means (5, 7, 9, 11, 13, 15) for producing a product (16) by coagulating matter to be removed in a fluid (2) to be treated, chemically catching and bonding the matter, and a filtering means (19, 20, 21) having an opening size through which the product (16) provided by the production means at a size larger than that of the matter can not pass, **characterized in that**
a fluid stirring means (13, 15) for stirring the fluid 2) to be treated is provided in said production means, and a treating means (74; 75) for at least sterilizing or oxidizing the matter to be removed in the fluid to be treated is provided in said fluid stirring means (13, 15).

5. A filtering and purifying system comprising a production means (5, 7, 9, 11, 13, 15) for producing a product (16) by coagulating matter to be removed in a fluid to be treated, chemically catching and bonding the matter, and a filtering means (19, 20, 21) having an opening size through which the product (16) provided by the production means at a size larger than that of the matter can not pass, **characterized in that**
a plurality of treating means (74) for at least sterilizing or oxidizing the matter to be removed in the fluid to be treated and a restoring means (85, 86, 89) for restoring reduced sterilizing or oxidizing functions of the treating means (74) are provided in said production means (13), and at least one or more of the treating means with the sterilizing or oxidizing functions restored function continuously during purifying operation.
